Europäisches Patentamt

European Patent Office

Office européen des brevets

(19)

(11) Veröffentlichungsnummer: **0 002 492**
**B1**

(12) **EUROPÄISCHE PATENTSCHRIFT**

(21) Anmeldenummer: 78101577.1

(22) Anmeldetag: 06.12.78

(51) Int. Cl.³: **C 07 C 11/02, C 07 C 1/20**

(54) Verfahren zur Herstellung von niederen Alkenen aus Methanol und/oder Dimethyläther

(30) Priorität: 10.12.77 DE 2755229

(43) Veröffentlichungstag der Anmeldung:
27.06.79 Patentblatt 79/13

(45) Bekanntmachung des Hinweises auf die Patenterteilung:
07.01.81 Patentblatt 81/01

(84) Benannte Vertragsstaaten:
BE DE FR GB IT NL

(56) Entgegenhaltungen:
DE - A - 2 615 150
US - A - 3 911 041
US - A - 3 979 472
US - A - 4 062 905
US - A - 4 079 095

(73) Patentinhaber: HOECHST AKTIENGESELL-
SCHAFT
Zentrale Patentabteilung Postfach 80 03 20
D - 6230 Frankfurt
Main 80 (DE)

(72) Erfinder: Wunder, Friedrich, Dr.
Jahnstrasse 46
D - 6093 Flörsheim am Main (DE)
Arpe, Hans-Jürgen, Dr.
Am Hirtengraben 19
D - 6233 Kelkheim (Taunus) (DE)
Hachenberg, Horst, Dr.
Mohnweg 1
D - 6229 Walluf (DE)
Leupold, Ernst Ingo, Dr.
Am Zäunefeld 15
D - 6392 Neu-Anspach (DE)

Courier Press, Leamington Spa, England.

Verfahren zur Herstellung von niederen Alkenen aus Methanol und/oder Dimethyläther

Es is bekannt, Methanol und/oder Dimethyläther bei Temperaturen oberhalb von etwa 260°C und Gesamtdrucken von 0,5 bis etwa 100 bar an speziellen Molsieben (Zeolithen) zu einem Gemisch verschiedener Alkane, Alkene und Aromaten umzusetzen. Dabei ist es z.B. nach der DE—OS 2.615.150 notwendig, nicht nur ein Molsieb nach zahlreichen physikalischen und chemischen Kriterien auszuwählen, sondern es müssen einige Maßnahmen getroffen werden — die jedoch die Leistung beeinträchtigen — wie Herabsetzen der Konversion, Herabsetzen des Methanol/Dimethyläther-Partialdrucks, Verdünnen des Molsiebs mit inertem Trägermaterial, damit eine brauchbare Olefin-Selektivität erreicht wird. Es entsteht aber im allgemeinen noch ein beträchtlicher Anteil an Aromaten.

Es ist weiterhin bekannt, daß Molsiebe mit Phosphor-Verbindungen modifiziert werden können — so z.B. nach US.PS 3.911.041 mit Trimethylphosphit — um damit die Selektivität der Methanol/Dimethyläther-Umsetzung bezüglich Alkenbildung zu erhöhen. Die Herstellung dieser mit Phosphor modifizierten Molsiebe ist aufwendig, sie verlangt wasserfreie Bedingungen und Einsatz kostspielige Phosphor-Verbindungen. Darüberhinaus ist die Lebensdauer dieser Molsieb-Katalysatoren verhältnismäßig kurz; sie müssen etwa alle drei Wochen regeneriert werden. Schließlich bleibt die mit derart modifizierten Molsieben erreichbare Selektivität an Olefinen doch noch unbefriedigend, es bilden sich immer noch beträchtliche Anteile an gesättigten und aromatischen Kohlenwasserstoffen.

Die Aufgabe der vorliegenden Erfindung besteht darin, die Umwandlung von Methanol und/oder Dimethyläther so zu gestalten, daß die technisch bedeutenden niederen Alkene wie Äthylen, Propen und Butene, besonders Äthylen, mit einer hohen Selektivität bei gleichzeitig hohem Umsatz von Methanol und/oder Dimethyläther erhalten werden. Das erfindungsgemäße Verfahren zur Herstellung niederer Alkene durch Umsetzung von Methanol und/oder Dimethyläther an Aluminiumsilikat-Katalysatoren ist dadurch gekennzeichnet, daß die Katalysatoren 0,1 bis 10 Gew.-% Mangan, bezogen auf das Aluminiumsilikat, enthalten.

Es hat sich weiterhin oft als vorteilhaft für eine hohe Selektivität erwiesen, zusätzlich zum Mangan noch weitere Elemente als Cokatalysatoren zu verwenden. Als solche sind Elemente geeignet, die in ihren Verbindungen ein-, zwei- oder dreiwertig vorkommen, wie beispielsweise die Alkalimetalle (insbesondere Lithium, Natrium, und Kalium), die Erdalkalimetalle (insbesondere Magnesium und Calcium), Zink, Lanthan, Seltene Erden (wie Praseodym, Neodym, Samarium, Gadolinium oder auch ihre Mischungen, wie im Didymium) und Beryllium. Von diesen Cokatalysatoren ist das Magnesium besonders bevorzugt.

Die Wirkungsweise des Mangans in Kombination mit einem Aluminiumsilikat ist für die Reaktion von Methanol und/oder Dimethyläther zu niederen Alkenen überraschend, da sich für eine selektive Umsetzung dieser Art bisher nur Phosphor- oder Stickstoffverbindungen zur Modifizierung von Molsieben als geeignet erwiesen haben.

Die im Benzin-Bereich und noch höher siedenden aliphatischen und aromatischen Kohlenwasserstoffe werden beim erfindungsgemäßen Verfahren nicht oder nur in verschwindend geringer Menge gebildet.

Darüberhinaus bringt die Anwesenheit von Mangan im Katalysator noch einen weiteren Vorteil mit sich. Bei den nach langer Betriebszeit des Katalysators im allgemeinen notwendigen Regenerierungen durch Abbrennen der Koksablagerungen mit Luft oder Sauerstoff sowie Wasserdampf wird durch die Redoxeigenschaften des Mangans der Oxidationprozeß erleichtert, so daß unter schonenden Bedingungen und ohne wesentliche Beeinflussung der Katalysatorstruktur, die Regenerierung erfolgen kann. Unter schonenden Bedingungen ist dabei zu verstehen, daß die Regenerierung bei gleichen Temperaturen schneller oder aber schon bei niedrigeren Temperaturen als nach dem Stand der Technik erfolgen kann.

Als Aluminiumsilikate kommen beispielsweise die üblichen, amorphen sauren Crackkatalysatoren in Frage, die im allgemeinen etwa 13 bis 25 Gew.-% Aluminiumoxid und 75 bis 87% Siliciumoxid enthalten. Zum weiteren sind auch natürliche oder synthetische kristalline Aluminiumsilikate geeignet, wie sie z.B. unter Bezeichnungen wie Faujasite, Zeolithe, Chabasite, Analcim, Gismondit, Gmelinit, Natrolith, Mordenite und Erionite oder auch allgemein als Molsiebe bekannt sind.

Bei den kristallinen Molsieben mit unterschiedlichen Porendurchmessern ist es zweckmäßig, solche mit großen Poren, z.B. Poren von 5 Å und höher zu verwenden.

Die Herstellung des erfindungsgemäßen Katalysators erfolgt durch Aufbringen von 0,1 bis 10 Gew.-% Mangan in Form von Mangansalzlösungen auf das Aluminiumsilikat. Dazu kann man beispielsweise das Aluminiumsilikat mit einer Lösung von Mangansalzen tränken und dann trocknen. Als Lösemittel kommen bevorzugt Wasser, Methanol, Formamid, Dimethylformamid oder auch deren Gemische in Frage. Im Allgemeinen ist Wasser bevorzugt. Auch durch längeres Einwirken einer Mangansalzlösung auf das Aluminiumsilikat und anschließendes Nachwaschen mit reinem Lösemittel und Trocknen kann Mangan aufgebracht werden. Bei Verwendung von Molsieben kann eine der bei diesen Materialien üblichen Methoden der Imprägnierung mit einem Metallkation gewählt werden; das kann ein Austausch der ursprünglich auf dem Molsieb vorhandenen Kationen gegen Mangan sein, es kann auch eine vorgelagerte Überführung des Molsiebes in die Protonenform mit anschließender Behandlung mit der Lösung eines Mangansalzes sein.

Die cokatalytisch wirksamen, weiteren Metallsalze können gleichzeitig mit dem Mangansalz aufgebracht werden, z.B. indem man eine Lösung von Mangansalz mit einer Lösung eines oder mehrerer der anderen Metallsalze mischt und dieses Gemisch einwirken läßt.

Sie können aber auch nacheinander auf das Aluminiumsilikat gebracht werden.

Als Mangansalze kommen alle löslichen Salze in Frage, z.B. Chlorid, Sulfat, Nitrat, Formiat, Acetat, Propionat, Butyrat, Lactat, Citrat, Tartrat und Salze der Apfelsäure. Entsprechendes gilt für die Cokatalysatoren. Verwendet man gemeinsame Lösungen von Mangan und cokatalytisch wirksamem Element, so ist die gegenseitige Löslichkeitsbeeinflussung zu berücksichtigen, d.h. bei Einsatz von Calcium oder Barium ist die Verwendung von Sulfat als Anion unzweckmäßig.

Zum weiteren ist bei Verwendung von natürlichen kristallinen Aluminiumsilikaten eine Wasserwäsche vor der Imprägnierung mit den Mangan- bzw. Cokatalysatorsalzen häufig empfehlenswert, damit nicht etwa die Metallsalze als Oxidhydrate vorzeitig gefällt werden können.

Die Katalysatoren werden nach der Imprägnierung bei Normaldruck, Vakuum oder Überdruck bei Normaltemperatur oder erhöhten Temperaturen getrocknet. Im allgemeinen liegen die Trocknungstemperaturen unterhalb von 600°C, vorzugsweise zwischen 100 und 200°C.

Bei Einsatz von Methanol als Ausgangsprodukt kann man entweder Methanol direkt über den erfindungsgemäßen Katalysator leiten oder aber es zunächst in einer vorgeschalteten Dehydratisierungsreaktion an einem üblichen Dehydratisierungskatalysator, wie Aluminiumoxid oder Aluminiumsilikat, in Dimethyläther überführen und diesen dann über den erfindungsgemäßen Katalysator leiten. Trotz der Zweistufigkeit der zuletzt gennanten Reaktionsführung können sich Vorteile dadurch ergeben, daß man einen Teil des bei der Gesamtreaktion abzutrennenden Wassers in dieser Vorstufe schon entfernt.

Man kann aber auch Gemische von Methanol und Dimethyläther oder Dimethyläther allein als Ausgangssubstanz verwenden.

Die Einsatzkomponenten Methanol und/oder Dimethyläther können auch mit Inertgasen verdünnt in die Reaktion eingesetzt werden. Zur Erniedrigung des Partialdruckes sind beispielsweise Stickstoff, Kohlendioxid, Alkene oder auch Wasser geeignet. Die Reaktion kann aber zu diesem Zweck auch bei vermindertem Druck bis herab zu 0,1 bar durchgeführt werden.

Bevorzugt ist jedoch die Durchführung bei Drucken von 1 bis 100 bar, besonders bevorzugt ein Druckbereich zwischen 1 und 50 bar.

Die Reaktionstemperatur liegt im allgemeinen zwischen 300 und 500°C, bevorzugt zwischen 350 und 450°C, besonders bevorzugt zwischen 380 und 420°C. Wählt man die Reaktionsbedingungen so, daß nur ein unvollständiger Umsatz an Methanol und/oder Dimethyläther erreicht wird, so können die umgesetzten Anteile angetrennt und zurückgeführt werden.

Die nach dem erfindungsgemäßen Verfahren hergestellen Alkene können nach üblichen Methoden, z.B. durch Destillation, von den als Nebenprodukt entstehenden Alkanen und voneinander getrennt werden.

Damit steht ein Verfahren zur Verfügung, das in besonders selektiver und damit wirtschaftlicher Weise die Herstellung von industriell bedeutenden niederen Alkenen aus Methanol und/oder Dimethyläther gestattet. Der erfindungsgemäße Katalysator kann in überraschend einfacher Weise aus leicht zugänglichen Substanzen hergestellt werden.

Die folgenden Beispiele erläutern das erfindungsgemäße Verfahren:

## Beispiel 1

Es werden 100 ml eines handelsüblichen Molsiebs mit der Bezeichnung 13x (Summenformel $Na_{86}(Al_2O_3)_{86}(SiO_2)_{106} \cdot 276\ H_2O$, Durchmesser der Porenöffnungen 10 Å, Wasseraufnahmekapazität 36%, Zeolith-Gitterstruktur) mit Wasser bei 25°C gewaschen, bis das überstehende Wasser einen pH von 7.3 erreicht; das nasse Molsieb wird in 100 ml gesättigter wäßriger Manganacetatlösung gegeben, 48 Stunden stehen gelassen, anschließend mit Wasser ausgewaschen und bei 120°C getrocknet. Dieser Katalysator enthält 4,4 Gew.-% Mangan. Über ihn werden 7 1 (berechnet auf Normzustand, d.h. 0°C und 1,013 bar) Dimethyläther pro Stunde bei Normaldruck und 380°C geleitet. Bei einem Umsatz von 54,9% erhält man 3,3 l eines Gemisches, das

31,6 Gew.-% Äthylen
29,1 Gew.-% Propylen
11,3 Gew.-% Butene
16,6 Gew.-% Methan
3,7 Gew.-% Äthan
1,6 Gew.-% Propan
6,1 Gew.-% Butan

enthält. Damit entstehen gesättigte und ungesättigte $C_2$-$C_4$-Kohlenwasserstoffe mit insgesamt 83,4% Selektivität. Die Selektivität zu Äthylen ist 31,6%, zu Propylen 29,1% und zu Buten 11,3%.

## Beispiel 2

200 ml eines 13x-Molsiebes in Strangform (Charakteristika s. Beispiel 1) werden in einem Rohr von 2,5 cm Durchmesser mit Wasser gewaschen, bis das überstehende Wasser einen pH von 7,0 erreicht. Anschließend läßt man innerhalb von 24 Stunden 400 ml einer mit Mangesiumpropionat und Manganbutyrat gesättigten wäßrigen Lösung durchlaufen und wäscht dann mit Wasser überschüssiges Mangan- und Magnesiumsalz aus, wobei ein Gehalt von 6,5 Gew.-% Mangan und 1,3 Gew.-% Magnesium erreicht wird. Nach dem Trocknen bei 150°C werden über diesen Katalysator 27,5 g/h Methanol bei 400°C und 1 bar geleitet. Bei einem Umsatz von 90,1% erhält man pro Stunde 11 g Wasser, 13 g Dimethyläther und 2,57 l (Normzustand) eines Kohlenwasserstoffgemisches, das

> 46,9 Gew.-% Äthylen
> 29,2 Gew.-% Propylen
> 5,3 Gew.-% Butene
> 12,3 Gew.-% Methan
> 3,7 Gew.-% Äthan
> 0,5 Gew.-% Propan
> 2,1 Gew.-% Butan

enthält. Da der gebildete Dimethyläther zurückgeführt werden kann, beträgt die Selektivität zu Äthylen 46,9%, zu Propylen 29,2% und zu Buten 5,3% d.h. diese drei Olefine entstehen mit insgesamt 81,5% Selektivität.

## Vergleichsbeispiel 1

Die Herstellung des Katalysators erfolgt wie in Beispiel 2, nur daß eine gesättigte Lösung verwendet wird, die lediglich Magnesiumpropionat und kein Manganbutyrat enthält. Über 200 ml dieses 13x-Molsiebes leitet man 27,5 g/h Methanol bei 1 bar und 400°C. Im Abgas befinden sich nur 0,1 Vol.-% Äthylen; der Rest ist Dimethyläther.

Bei 500°C steigt der Äthylengehalt auf 1,3%, um bei 600°C wieder unter 0,1% abzufallen.

## Vergleichsbeispiel 2

Über 200 ml eines 13x-Molsiebes (Charakteristika s. Beispiel 1) werden ohne jede Vorbehandlung 27,5 g/h Methanol bei 1 bar und 400°C geleitet. Das Abgas enthält kein Äthylen, sondern fast nur Dimethyläther neben Spuren von Kohlenmonoxid und Wasserstoff.

## Vergleichsbeispiel 3

Man verfährt wie in Vergleichsbeispiel 2, nur daß das 13x-Molsieb vor seinem Einsatz mit Wasser gewaschen wird, bis das Waschwasser neutral ist. Das Abgas hat dieselbe Zusammensetzung wie in Vergleichsbeispiel 2.

## Beispiel 3

200 g (=500 ml) eines handelsüblichen amorphen Aluminiumsilikats (25 Gew.-% $Al_2O_3$, 74,5 Gew.-% $SiO_2$, 0,05 Gew.-% Na, 0,03 Gew.-% Fe, 0,03 Gew.-% Ca, BET-Oberfläche 325 $m^2/g$, Porenvolumen 0,45 ml/g) werden (entsprechend seinem Porenvolumen) mit einer aus 23,2 g Manganformiat und 77 ml Wasser bestehenden Mangansalzlösung imprägniert und bei 120°C getrocknet. Der Mangangehalt des Katalysators beträgt 3,8 Gew.-%. Über diesen Katalysator werden bei 380°C und 0,5 bar 46 g/h Methanol geleitet. Es entstehen 13,5 l (Normzustand) Abgas je Stunde, das

> 18,0 Gew.-% Äthylen
> 19,9 Gew.-% Propylen
> 12,2 Gew.-% Butene
> 11,6 Gew.-% Methan
> 2,0 Gew.-% Äthan
> 1,1 Gew.-% Propan
> 23,2 Gew.-% Butan
> 0,6 Gew.-% Kohlenmonoxid
> 0,04 Gew.-% Wasserstoff
> 11,4 Gew.-% Dimethyläther

enthält. Es fallen 26 g Kondensatwasser mit 13,3 Gew.-% Methanol an. Dies entspricht einem Umsatz von 92,5% und einer Selektivität zu gesättigten und ungesättigten Kohlenwasserstoffen mit

> $C_1$ von 13,1%
> $C_2$ von 22,7%
> $C_3$ von 23,7%
> $C_4$ von 40, 0%

wenn unumgesetztes Methanol und gebildeter Dimethyläther zuruckgeführt werden. Die $C_2$-$C_4$-Kohlenwasserstoffe gesättigte und ungesättigte) entstehen mit einer Selektivität von insgesamt 86,4%, die Selektivität zu $C_2$-$C_4$-Olefinen beträgt 58,3%.

## Beispiel 4

Über 300 ml eines handelsüblichen engporigen (low pore diameter) Aluminiumsilikat-Katalysators (15,4 Gew.-% $Al_2O_3$, 0,028 Gew.-% $Fe_2O_3$, 0,007 Gew.-% $Na_2O$, 84,5 Gew.-% $SiO_2$, BET-Oberfläche 485 $m^2/g$, Porenvolumen 0,55 ml/g), der mit einer 20%igen wäßrigen Manganacetatlösung behandelt worden war und 1,3 Gew.-% Mangan enthält, werden bei 500°C und 15 bar 200 g/h Methanol geleitet. Man erhält ein Reaktionsgas, das

> 8,3 Gew.-% Methanol
> 1,6 Gew.-% Dimethyläther
> 14,1 Gew.-% Äthylen
> 18,2 Gew.-% Propylen
> 5,0 Gew.-% Butene
> 1,9 Gew.-% Methan
> 50,9 Gew.-% Wasser

enthält. Der Umsatz an Methanol beträgt 91,7%, die Selektivität zu Kohlenwasserstoffen (gesättigte und ungesättigte) ist 89,4%, die Selektivität zu Äthylen 35,0%, zu Propylen 45,6% und zu Buten 12,5%. Die $C_2$-$C_4$-Olefine entstehen zusammen mit einer Selektivität von 93,1%.

### Beispiel 5

Es werden 50 ml eines Handelsüblichen (Handelsname AGZ 50) Molsieb-Katalysators (30 Gew.-% $Al_2O_3$, 2,57 Gew.-% Seltenerdoxide, 0,49 Gew.-% $SO_4$=0,29 Gew.-% $Na_2O$, 66,6 Gew.-% $SiO_2$, BET-Oberfläche nach 3 Stunden Erhitzen auf 1000°F: 290 m²/g, Porenvolumen 0,43 ml/g, mittlere Schüttdichte = average bulk density ABD 0,6 g/ml, mittlere Teilchengröße = average particle size APS 68$\mu$) mit einer gesättigten wäßrigen Lösung von Manganchlorid und Magnesiumchlorid 10 Stunden gerührt, dann mit Wasser gewaschen und bei 110°C getrocknet. Der Mangangehalt des Katalysators beträgt 0,5 Gew.-%, der Gehalt an Magnesium 0,1 Gew.-%. Über diesen Katalysator werden 20 l (Normzustand) Dimethyläther je Stunde bei 390°C und 1,5 bar geleitet. Das Reaktionsprodukt enthält

21,6 Gew.-% Äthylen
19,3 Gew.-% Propen
14,4 Gew.-% Butene
3,3 Gew.-% Methan
3,8 Gew.-% Dimethyläther
37,6 Gew.-% Wasser.

Der Umsatz beträgt 96,2%, die Selektivität zu Äthylen 36,9%, zu Propylen 32,9%, zu Butenen 24,6%. Die $C_2$-$C_4$-Olefine entstehen mit einer Selektivität von insgesamt 94,4%.

### Beispiel 6

600 g eines Molsiebs 13x werden in ein senkrecht stehendes Rohr von 25 mm Innendurchmesser gefüllt und mit entsalztem Wasser gewaschen. Dem letzten Waschwasser wird Kohlendioxid zugegeben, und dann wird so lange gewaschen, bis das $CO_2$-haltige Waschwasser nach 5 Stunden Kontaktzeit mit dem Molsieb einen pH von 6,8 hat. Dann wird innerhalb von 48 Stunden eine konzentrierte wäßrige Lösung von Mangan- und Magnesiumacetat (2, 5 l) durchgeleitet. Anschließend wird mit destilliertem Wasser gewaschen, bis keine Manganionen im Waschwasser mehr nachweisbar sind. Der Katalysator enthält 6,8 Gew.-% Mangan und 1,3 Gew.-% Magnesium. Über diesen Katalysator werden bei 430°C und 25 bar stundlich 2,8 l (=2,2 kg) Methanol geleitet. Das Reaktionsprodukt hat folgende Zusammensetzung:

14,3 Gew.-% Äthylen
12,4 Gew.-% Propen

7,2 Gew.-% Butene
3,3 Gew.-% Methan
1,9 Gew.-% Äthan
3,1 Gew.-% Butan
55,1 Gew.-% Wasser
1,2 Gew.-% Methanol
0,8 Gew.-% Dimethyläther.

| | |
|---|---|
| Der Methanol-Umsatz beträgt | 98,8% |
| Die Selektivität zu (gesättigten und ungesättigten) Kohlenwasserstoffen | 97,3% |
| Die Selektivität zu Äthylen | 33,3% |
| zu Propen | 28,9% |
| zu Buten | 16,8% |
| zu Methan | 7,7% |
| zu Äthan | 4,4% |
| zu Butan | 7,2% |
| Die Selektivität zu $C_2$-$C_4$-Kohlenwasserstoffen (gesättigte und ungesättigte) | 90,6% |
| Die Selektivität zu $C_2$-$C_4$-Olefinen | 79,0% |

**Patentansprüche:**

1. Verfahren zur Herstellung niederer Alkene durch Umsetzung von Methanol und/oder Dimethyläther an Aluminiumsilikat-Katalysatoren, dadurch gekennzeichnet, daß die Katalysatoren 0,1 bis 10 Gew.-% Mangan, bezogen auf das Aluminiumsilikat, enthalten.

2. Verfahren nach Anspruch 1, dadurch gekennzeichnet, daß die Katalysatoren Elemente der 1. oder 2. Hauptgruppe oder der 2. oder 3. Nebengruppe als Cokatalysatoren enthalten.

3. Verfahren nach Anspruch 1 und 2, dadurch gekennzeichnet, daß die Katalysatoren Magnesium als Cokatalysator enthalten.

4. Verfahren nach Ansprüchen 1 bis 3, dadurch gekennzeichnet, daß die Aluminiumsilikate kristallin sind.

5. Verfahren nach Ansprüchen 1 bis 4, dadurch gekennzeichnet, daß der Reaktionsdruck 1-100 bar beträgt.

6. Verfahren nach Anspruch 1 bis 5, dadurch gekennzeichnet, daß die Reaktionstemperatur 300-500°C beträgt.

**Revendications**

1. Procédé de fabrication d'alcènes inférieurs par conversion du méthanol et/ou de l'éther diméthylique sur des catalyseurs à base de silicates d'aluminium, procédé caractérisé en ce que les catalyseurs employés contiennent 0,1 à 10% en poids de manganèse par rapport au silicate d'aluminium.

2. Procédé selon la revendication 1, caractérisé en ce que les catalyseurs contiennent aussi comme cocatalyseurs des éléments du groupe principal 1 ou 2 ou des sous-groupes 2 et 3 de la Classification Périodique.

3. Procédé selon la revendication 1 ou 2, caractérisé en ce que les catalyseurs contiennent du magnésium comme cocatalyseur.

4. Procédé selon l'une quelconque des revendications 1 á 3, caractérisé en ce que les silicates d'aluminium sont cristallisés.

5. Procédé selon l'une quelconque des revendications 1 à 4, caractérisé en ce que la pression de réaction est de 1 à 100 bars (0,1 à 10 MPa).

6. Procédé selon l'une quelconque des revendications 1 à 5, caractérisé en ce que température de réaction est de 300 à 500°C.

## Patent Claims:

1. Process for the manufacture of lower alkenes characterized by reacting methanol and/or dimethyl ether on aluminum silicate catalysts containing from 0.1 to 10% by weight of manganese, calculated on the aluminum silicate.

2. The process of claim 1, wherein the catalyst contains in addition elements of the 1st or 2nd main group or of the 2nd or 3rd subgroup of the Periodic Table as co-catalysts.

3. The process of claim 1, wherein the catalyst contains in addition magnesium as cocatalyst.

4. The process of claim 1, wherein cristalline aluminum silicate is used as catalyst.

5. The process of claim 1, wherein the reaction pressure is from 1 to 100 bars.

6. The process of claim 1, wherein the reaction temperature is from 300 to 500°C.